# EUROPÄISCHE PATENTSCHRIFT

(11) **EP 1 033 975 B1**
(45) Veröffentlichungstag und Bekanntmachung des Hinweises auf die Patenterteilung: **20.03.2002**
(21) Anmeldenummer: 98965661.6
(22) Anmeldetag: 09.11.1998
(51) Int. Cl.: A61K 9/14

(54) **VERFAHREN ZUR HERSTELLUNG VON LÖSUNGSMITTELFREIEN NICHT-KRISTALLINEN BIOLOGISCH AKTIVEN SUBSTANZEN**
METHOD FOR PRODUCING SOLVENT-FREE NON-CRYSTALLINE BIOLOGICALLY ACTIVE SUBSTANCES
PROCEDE PERMETTANT DE FABRIQUER DES SUBSTANCES BIOLOGIQUEMENT ACTIVES, DEPOURVUES DE SOLVANTS ET NON CRISTALLINES

(30) Priorität: 28.11.1997 DE 19752895
(43) Veröffentlichungstag der Anmeldung: 13.09.2000
(73) Patentinhaber: KNOLL AKTIENGESELLSCHAFT, 67061 Ludwigshafen (DE)
(72) Erfinder: BREITENBACH, Jörg, D-68199 Mannheim (DE); REINHOLD, Ulrich, D-67346 Speyer (DE); ZEIDLER, Jürgen, D-67112 Mutterstadt (DE); ROSENBERG, Jörg, D-67158 Ellerstadt (DE)
(74) Vertreter: Schweiger, Georg, Dr.
(86) Internationale Anmeldenummer: EP9807141
(87) Internationale Veröffentlichungsnummer: WO9927891

(56) Entgegenhaltungen:
- EP-A- 0 665 009
- EP-A- 0 813 904
- WO-A-95/01321
- WO-A-97/18839
- DE-A- 3 310 676

## Beschreibung

Die vorliegende Erfindung betrifft ein Verfahren zur Herstellung von lösungsmittelfreien nicht-kristallinen biologisch aktiven Substanzen aus den entsprechenden lösungsmittelhaltigen kristallinen Substanzen in einem Extruder.

Häufig ist ein und dieselbe chemische Substanz in der Lage, Festkörper auszubilden, denen verschiedene Ordnungszustände zugrunde liegen. Dieser als Polymorphismus bezeichnete Zustand läßt sich ebenfalls durch das erfindungsgemäße Verfahren beeinflussen. Zu den unterschiedlichen kristallographischen Formen gehören auch pseudo-polymorphe Formen. Diese sind solche, an deren Aufbau nicht nur die betreffende Substanz, sondern auch Fremdsubstanzen durch Ausbildung von Mischkristallisaten, Solvaten oder Hydraten beteiligt sind. Mischkristallisate sind Kristallisate, aus zwei oder mehreren chemischen Stoffen, wobei die beteiligten Komponenten eine spezielle gemeinsame kristallographische Struktur aufbauen. Solvate und Hydrate sind als Sonderfälle aufzufassen, bei denen eine der Mischungskomponenten ein Lösungsmittel darstellt, wobei mit Wasser Hydrate gebildet werden.

Hydrate sind von besonderer Bedeutung. Sie bauen sich aufgrund der strengen Strukturbedingungen stöchiometrisch auf und zeigen oft ein signifikant schlechteres Lösungsverhalten als die wasserfreien Formen, da das Kristallwasser zusätzliche Sekundärvalenzen im Molekülverband abzusättigen vermag. Diese können unter Umständen aus sterischen Gründen in der wasserfreien Form keine Absättigung finden. Dadurch sind kristallwasserhaltige Formen thermodynamisch oftmals stabiler.

Aufgrund ihres Herstellungsverfahrens enthalten viele biologisch aktive Substanzen Lösungsmittel.

Die Entfernung solcher Lösungsmittel, insbesondere der im Kristall gebundenen Lösungsmittel,durch herkömmliche Verfahren wie Erhitzen oder Gefriertrocknen bereitet jedoch häufig, Schwierigkeiten.

Insofern ist es von Interesse , ein Verfahren zur Verfügung zu stellen mit dem biologisch aktive Substanzen in einem einfachen Prozeß in eine lösungsmittelfreie Form überführt werden.

Weiterhin ist bekannt, daß Substanzen in ihrer kristallinen Form häufig eine schlechtere Bioverfügbarkeit aufweisen, als die entsprecherde amorphe Form.

Aus der EP-A 0 665 009 ist ein Verfahren bekannt, bei dem man Wirkstoffe in einem Zwei-Schnecken-Extruder von einem morphologischen Zustand in einen anderen überführt. Dabei ist jedoch weder der Einsatz von Unterdruck offenbart, noch gibt das Verfahren Anleitung dazu, wie mit Wirkstoffen die Kristallwasser oder Lösungsmittel enthalten, zu verfahren ist.

Aufgabe der vorliegenden Erfindung war es ein verfahren zur Herstellung von lösungsmittelfreien nicht-kristallinen, also amorphen, biologisch aktiven Substanzen aus den entsprechenden lösungsmittelhaltigen kristallinen biologisch aktiven Substanzen zur Verfügung zu stellen.

Demgemäß wurde ein Verfahren zur Herstellung von nicht-kristallinen biologisch aktiven Substanzen gefunden, welches dadurch gekennzeichnet ist, daß man die kristallinen lösungsmittelhaltigen Substanzen im Extruder und in Abwesenheit von Hilfsstoffen aufschmilzt, wobei in einer Zone des Extruders ein Vakuum angelegt wird.

Erfindungsgemäß können dazu, neben pharmazeutischen Wirkstoffen, eine Vielzahl von biologisch aktiven Substanzen eingesetzt werden, die naturgemäß Lösungsmittel in ihrem Kristallgitter gebunden haben oder aber lediglich mit einem Lösungsmittel befeuchtet sind.

Das erfindungsgemäße Verfahren eignet sich beispielsweise zur Formulierung folgender Stoffe oder deren physiologisch akzeptablen Salzen
- Antiinfektiva
   Aciclovir, Aminoglykoside, Amphotericin B, Azol-Antimykotika, Clotrimazol, Itraconazol, Sepraconazol, Clindamycin, Cephalosporine, Chloramphenicol, Erythromycin, 5-Fluoruracil, Etoposid, Flucytosin, Ganciclovir, Griseofulvin, Gyrasehemmstoffe, Isoniacid, Lincosamide, Mebendazol, Mefloquin, Metronidazol, Mitroimidazole, Novobiocin, Platinverbindungen, Polymyxin B, Praziquantel, Pyrimethamin, Rifampicin, Saquinavir, Streptomycin, Sulfonamide, Tetracycline, Trimethoprim, Vancomycin, Zidovudin;
- Antipyretika, Analgetika, antiinflammatorische Mittel, Paracetamol, Ibuprofen, Ketoprofen, Oxaprozin, Acetylsalicylsäure, Morphin, Propoxyphen, Phenylbutazon;
- Antibiotika
   Rifampicin, Griseofulvin, Chloramphenicol, Cycloserin, Erythromycin, Penicilline wie Penicillin G, Streptomycin, Tetracyclin;
- Antiepileptika
   Hydantoine, Carbamazepin;
- Antitussiva und Antiasthmatika
   Diphenhydramin;
- Antirheumatika
   Chloroquin, Indomethacin, Goldverbindungen, Phenylbutazon, Oxyphenylbutazon, Penicillinamin;
- Hypnotika
   Barbiturate, Phenobarbital, Zolpidem, Dioxopiperidine, Ureide;
- Insektizide
   Aldrin, Dieldrin, Chlorphenotan, Hexachlorcyclohexan;
- Herbizide
   Vinclozolin, Strobilurine;
- Psychopharmaka, Neuroleptika
   Perazin, Promazin, Sulpirid, Thioridazin, Chlorpromazin, Meprobamat, Triflupromazin, Melperon, Clozapin, Risperidon, Reserpin;
- Tranquillantien;
- Antidepressiva
   Imipramin, Paroxetin, Viloxazin, Moclobemid;
- Psychotonika;
- Psychomimetika;
- Diuretika
   Kaliumcanrenoat, Schleifendiuretika, Furosemid, Hydrochlorothiazid, Spironolacton, Thiazide, Triamteren;
- Hormone
   Androgene, Antiandrogene, Gestagene, Glucocorticoide, Oestrogene, Cortisol, Dexamethason, Prednisolon, Testosteron, Adiuretin, Oxytocin, Somatropin, Insulin;
- Immunsuppresiva
   Ciclosporin;
- Bronchodilatoren;
- Muskelrelaxantien, Tranquillantien
   Carisoprodol, Tetrazepam, Diazepam, Chlordiazepoxid;
- Enzyme
   Lipase, Phytase;
- Gichtmittel
   Allopurinol, Colchicin;
- Antikoagulatien
   Cumarine;
- Antiepileptika
   Phenotoin, Phenobarbital, Primidon, Valproinsäure, Carbamazepin;
- Antihistaminika
   Chlorphenoxamin, Dimenhyrinat;
- Antimimetika;
- Antihypertonika, Antiarrhythmika
   Lidocain, Procainamid, Chinidin, Calciumantagonisten, Glyceroltrinitrat, Isosorbiddinitrat, Isosorbid-5-mononitrat, Pentaerythrityltetranitrat, Nifedipine, Diltiazem, Felodipin, Verapamil, Reserpin, Minoxidil, Reserpin, Captopril, Enalnapril, Lisinopril;
- Sympathomimetika
   Norfenefrin, Oxedrin, Midodrin, Phenylephrin, Isoprenalin, Salbutamol, Clenbuterol, Ephedrin, Tyramin, Isoprenalin, β-Blocker wie Alprenolol, Metoprolol, Bisoprolol;
- Antidiabetika
   Biguanide, Sulfonylharnstoffe, Carbutamid, Tolbutamid, Glibenclamid, Metformin, Acarbose, Troglitazon;
- Eisenpräparationen;
- Vitamine
   Vitamin C, B, A, D, Folsäure;
- ACE-Hemmer
   Captopril, Ramipril, Enalapril;
- Anabolika;
- Iod-Verbindungen;
- Röntgenkontrastmittel;
- ZNS-aktive Verbindungen;
- Antiparkinsonmittel
   Biperiden, Benzatropin, Amantadin, opioide Analgetika, Barbiturate, Benzodiazepine, Disulfiram, Lithiumsalze, Theophyllin, Valproinat, Neuroleptika;
- Zycostatika;
- Antispasmolytika;
- Vasodilatoren
   Naftidrofuryl, Pentoxifyllin.

Weiterhin ist es möglich, Gemische von biologisch aktiven Substanzen einzusetzen. Amorphe Feststoffe haben wie Flüssigkeiten ein hohes Lösungsvermögen für Mischungspartner. Deshalb ist es möglich, daß eine biologisch aktive Substanz eine weitere in der Schmelze löst. Dies kann zu Mischungsdiagrammen mit Eutektika führen. Je nach Lage der Schmelzkurven kann die eine oder andere Substanz kristallin neben der amorphen ersten vorliegen.

Bevorzugte Substanzen sind solche, die in der Liste aufgeführt sind oder solche, die organische Lösungsmittel und/oder Wasser eines Kristallisationsprozessen enthalten. Dies gilt auch für Vitamine.

Bevorzugte Wirkstoffe für das erfindungsgemäße Verfahren sind Amilorid-HCl · 2 H₂O, Amoxicillin · 3 H₂O, Ampicillin . 3 H₂O, Atropinsulfat · 1 H₂O, Benzylpenicillin-Benzathin · 1 H₂O, Calciumfolinat 5 H₂O, Carbidopa . 1 H₂O, Cefepim · 1 H₂O, Cefixim · 3 H₂O, Ceftazidim · 5 H₂O, Cephaclor · 1 H₂O, Cephalexin · 1 H₂O, Chinidinhydrogensulfat · 4 H₂O, Chininsulfat · 2 H₂O, Clindamycin-HCl · H₂O, Codeinphosphat · 1/2 H₂O, Cyclophosphamid · 1 H₂O, Dihydralazinsulfat · 21/2 H₂O, Doxycyclin · 1 H₂O, Doxycyclin · 1/2 C₂H₆OH · 1/2 H₂O, Eisen(II)gluconat · 2 H₂O, Eisen(I)sulfat · 1 H₂O, Flucloxacillin-Natrium · 1 H₂O, g-Strophanthin · 8 H₂O, Ipratropiumbromid . 1 H₂O, Lidocain-HCl · 1 H₂O, Lincomycin-HCl · 1 H₂O, Loracarbef · 1 H₂O, Mepacrin-2 HCl · 2 H₂O, Metamizol-Natrium · 1 H₂O, Methyldopa · 11/2 H₂O, Minocyclin-HCl · 2 H₂O, Morphinsulfat · 5 H₂O, Natriumibuprofenat · 2 H₂O, Natriumpicosulfat · 1 H₂O, Oxyphenbutazon · 1 H₂O, Pyritinol-2HCl · 1 H₂O, Sultamicillintosilat · 2 H₂O, Theophyllin-Ethylendiamin · 2 H₂O oder Vinblastinsulfat · 1 H₂O oder deren Gemische.

Die Menge an Lösungsmittel kann dabei in einem weiten Bereich schwanken. So sind Wirkstoffe aus der pharmazeutischen Anwendung bekannt, die niedrige Gehalte von Lösungsmittel von < 1 Gew.-% auf weisen. Bei der chemischen Synthese der Wirkstoffe kann es trotz Trocknung aber zur Befeuchtung der biologisch aktiven Substanz kommen, so daß Lösungsmittelgehalte von mehr als 10 Gew.-% gefunden werden.

Die Herstellung der erfindungsgemäßen biologisch aktiven Substanzen erfolgt unter Einsatz von Scherkräften und Zufuhr thermischer Energie. Bevorzugt erfolgt das Mischen in einem Ein- oder Mehr-Schnecken-Extruder, besonders bevorzugt einem Zwei-Schnecken-Extruder. Durch Zufuhr thermischer Energie wird eine Schmelze der biologisch aktiven Substanz erzeugt. Dies geschieht üblicherweise durch Aufheizen des Extrudermantels auf Temperaturen im Bereich von 35 - 350°C. Die Prozeßtemperatur richtet sich nach dem Schmelzpunkt der Substanz oder des Substanzgemisches.

Die geschmolzene biologisch aktive Substanz oder die Mischung der geschmolzenen biologisch aktiven Substanzen wird durch die Schneckenbewegung in Richtung auf den Extruderauslass, der vorzugsweise aus einer Düse besteht, gefördert. Erfindungsgemäß kann die Extrusion aber auch aus dem offenen Extruder heraus erfolgen. Dann kommt es im letzten Schuß zu einem Druckabfall.

In Abhängigkeit von der Viskosität der Schmelze und der Schneckengeometrie kann der Betriebsdruck in den Extruderzonen, die vor der mit einem Vakuumansatz ausgerüsteten Zone liegen wie Extruderkanal 1 bis 500 bar betragen. Bevorzugt sind Drücke von 3 bis 60 bar.

Erfindungsgemäß wird bevorzugt im letzten Segment oder Schuß (Zone des Extruders) vor der Düse oder dem Extruderkanalende ein Vakuum von 10 bis 600 mbar bevorzugt 30 - 400 mbar. besonders bevorzugt 50 - 100 mbar, angelegt. In diesem Vakuumsegment wird das Lösungsmittel aus der Masse entzogen. Es entsteht eine amorphe Masse, die durch Abkühlen in einem weiteren Segment und Einsatz von Scherkräften, die z. B. über besondere Schneckenkonfigurationen eingetragen werden können, zu einem Pulver zerkleinert werden kann. Man kann also auch die Schmelze austragen, dann abkühlen und anschließend mahlen.

Solche Pulver biologisch aktiver Substanzen sind geeignet für pharmazeutische und kosmetische Zubereitungen aber auch für den Einsatz im Food, Feed und Veterinärbereich.

Die biologisch aktive Substanz liegt besonders bevorzugt als amorphes Pulver vor.

Die Beurteilung, in welchem Zustand pulverförmiges Material vorliegt, kann eindeutig nur mit Hilfe der Röntgenfeinstruktur-Untersuchung durchgeführt werden. Den Untersuchungsmethoden liegt die Tatsache zugrunde, daß beim Durchstrahlen einer geordneten Materie mit Röntgenlicht Interferenzerscheinungen auftreten. Mit Hilfe thermoanalytischer Methoden wie der Differenzthermoanalyse und der Differentialscanningkalorimetrie lassen sich Phasentransformationen, d. h. Veränderungen im Zustand der Materie in Abhängigkeit von der Temperatur, beobachten. Sie geben wichtige Hinweise über mögliches Verhalten eines Wirkstoffs während der Verarbeitung.

In der Heizzone des Extruders, in der das Mischen und das Aufschmelzen erfolgt, kann die Schneckengeometrie dicht-kämmend, kämmend oder nicht-kämmend gewählt werden, wobei eine dicht-kämmende Schnecken-Geometrie bevorzugt ist. Die Schnecken können sich gegenläufig oder bevorzugt gleichsinnig drehen. Im Mischund Schmelzbereich sind neben Förderelementen auf den Schnecken bevorzugt Misch- und Knetelemente angeordnet. Förderelemente sind ein- und mehrgängige Schneckenelemente unterschiedlicher Steigung. Mischelemente sind zahnradähnliche Zahnscheibenelemente oder mit Durchbrüchen versehene rückwärtsfördernde Elemente, wobei die Durchbrüche teilweise bis zum Schneckenkern reichen können oder zumindest die Hälfte des Helixradius ausmachen. Knetelemente sind Zwei- oder Dreispitzscheiben, wobei die Elemente immer mehrere Scheiben mit unterschiedlicher Breite und unterschiedlichen Versatzwinkel zueinander besitzen. Der Mantel der Kühlzone wird mit flüssigem Kühlmedium gekühlt. In der Förderzone der Kühlzone wird die Temperatur bevorzugt auf 5° - 30° Celsius unter die Erweichungstemperatur der zu kühlenden Masse eingestellt. Über die gesamte Kühlzone kann die Temperatur in Fließrichtung je nach Erweichungspunkt der Massen um bis zu 150° Celsius unter den Erweichungspunkt abgesenkt werden. Es kann sich auch empfehlen, im Mischbereich der Kühlzone eine Schockkühlung vorzunehmen, und den Mantel auf Temperaturen im Bereich von -10° Celsius bis +10° Celsius zu kühlen.

Die detaillierte Schneckengeometrie richtet sich auch nach dem Schmelzpunkt der zu verarbeitenden Substanzen.

Mit Hilfe des erfindungsgemäßen Verfahrens lassen sich Massen mit Korngrößen im Bereich von 0,0001 bis 50mm Durchmesser herstellen. Je nach Wahl der Schneckendurchmesser, der Misch- und Knetelemente und der Schneckendrehzahlen erhält man großkörnige Partikel (10 - 50mm), mittelkörnige (1 - 3mm), kleinkörnige (0,3 - 1mm), feinkörnige (0,1 - 0,3mm), dichtkörnige (0.03 - 0,1mm) oder mikrokristalline (0,001 - 0,03mm) Partikel. Bevorzugt werden Korngrößen von 0,001 - 10mm, besonders bevorzugt 0,005 - 3mm. Welche Korngrößen man im einzelnen einstellt, richtet vor allem nach den gewünschten Anwendungsbereich. Die partikulären Zubereitungen weisen eine gute Einheitlichkeit in der Korngrößenverteilung auf, so daß sie ohne weitere Siebvorgänge weiter verarbeitet werden können.

Die erfindungsgemäßen Massen können zu Tabletten, Zäpfchen, Granulaten, Trinkgranulaten, Pellets, Implantaten, Schwimmtabletten verarbeitet werden oder als Kapselfüllung dienen.

### Herstellbeispiele

Für die Herstellung der Extrudate wurde ein gleichsinnig drehender, kämmender Doppelschneckenextruder (ZSK 30 der Firma Werner & Pfleiderer, Stuttgart, Deutschland) verwendet, welcher aus 8 separat heiz- bzw. kühlbaren kammerartigen Zonen bestand. Im folgenden werden die Zonen als "Zone 1", "Zone 2", usw. bezeichnet, wobei die Ausgangssubstanz bei Zone 1 eintritt und bei Zone 8 austreten soll. Zone 1 wurde mit Wasser gekühlt (Temperatur des ablaufenden Wassers: 30° Celsius). Die Zone 2 wurde während der gesamten Versuchsdauer bei 200° Celsius, die Zonen 3 und 4 bei 210° Celsius betrieben. Die Zone 4 enthielt einen Vakuumvorstoß an dem ein Vakuum mit 150 millibar angelegt war. Die Zonen 5, 6, 7 bzw. 8 wurden mit Wasser gekühlt, wobei sich Kühlwasserablauftemperaturen von 40° Celsius, 30° Celsius, 25° Celsius bzw. 20° Celsius ergaben.

### Beispiel 1

### Herstellung eines amorphen Natriumibuprofenats

In Zone 1 dieses Extruders wurde bei einer Umdrehungszahl von 36 Umdrehungen pro Minute stündlich 1700 g Natriumibuprofenatdihydrat über mit Schnecken ausgerüstete Dosierbandwagen eingetragen. Der Extruder wurde 10 Stunden betrieben. Das Ibuprofenat lag anschließend als amorphe wasserfreie Masse vor.

### Beispiel 2

Aus einem Kristallisationsprozeß erhaltenes Natriumibuprofenat mit einem Gehalt von 2 Gew.-% Wasser und 2 Gew.-% Methanol wurde analog Beispiel 1 verarbeitet. Es wurde ein amorphes lösungsmittelfreies Pulver erhalten.

## Patentansprüche

1. Verfahren zur Herstellung von lösungsmittelfreien nicht-kristallinen biologisch aktiven Substanzen aus den entsprechenden kristallinen biologisch aktiven Substanzen in einem Extruder, **dadurch gekennzeichnet, daß** man die lösungsmittelhaltigen Substanzkristalle im Extruder unter Vakuum und in Abwesenheit von Hilfsstoffen aufschmilzt, wobei in einer Zone des Extruders ein Vakuum angelegt wird.

2. Verfahren nach Anspruch 1, **dadurch gekennzeichnet, daß** man das Verfahren unter Inertgas betreibt.

3. Verfahren nach Anspruch 2; **dadurch gekennzeichnet, daß** das Inertgas Stickstoff ist.

4. Verfahren nach Anspruch 1, **dadurch gekennzeichnet, daß** das Lösungsmittel im Kristallgitter der biologisch aktiven Substanz eingebunden ist.

5. verfahren nach Anspruch 4, **dadurch gekennzeichnet, daß** das Lösungsmittel Wasser ist.

6. Verfahren nach Anspruch 4, **dadurch gekennzeichnet, daß** das Lösungsmittel ein organisches Lösungsmittel ist.

7. Verfahren nach Anspruch 1, **dadurch gekennzeichnet, daß** man mehr als eine biologisch aktive Substanz einsetzt.

## Claims

1. A process for preparing solvent-free noncrystalline bioactive substances from the corresponding crystalline bioactive substances in an extruder, wherein the solvent-containing substance crystals are melted in the extruder under reduced pressure and in the absence of auxiliaries, the pressure being reduced in one zone of the extruder.

2. A process as claimed in claim 1, which is carried out under inert gas.

3. A process as claimed in claim 2, wherein the inert gas is nitrogen.

4. A process as claimed in claim 1, wherein the solvent is integrated into the crystal lattice of the bioactive substance.

5. A process as claimed in claim 4, wherein the solvent is water.

6. A process as claimed in claim 4, wherein the solvent is an organic solvent.

7. A process as claimed in claim 1, wherein more than one bioactive substance is employed.

## Revendications

1. Procédé pour la préparation de substances biologiquement actives, exemptes de solvants et non-cristallisées, à partir des substances biologiquement actives cristallisées correspondantes dans une extrudeuse, **caractérisé par le fait qu'**on fait fondre les cristaux de substance contenant du solvant dans l'extrudeuse sous vide et en l'absence d'adjuvants, tandis qu'un vide est appliqué dans une zone de l'extrudeuse.

2. Procédé selon la revendication 1, **caractérisé par le fait qu'**on conduit le procédé sous gaz inerte.

3. Procédé selon la revendication 2, **caractérisé par le fait que** le gaz inerte est l'azote.

4. Procédé selon la revendication 1, **caractérisé par le fait que** le solvant est intégré dans le réseau cristallin de la substance biologiquement active.

5. Procédé selon la revendication 4, **caractérisé par le fait que** le solvant est l'eau.

6. Procédé selon la revendication 4, **caractérisé par le fait que** le solvant est un solvant organique.

7. Procédé selon la revendication 1, **caractérisé par le fait qu'**on utilise plus d'une substance biologiquement active.
